# EUROPEAN PATENT SPECIFICATION

(11) **EP 3 261 625 B1**
(45) Date of publication and mention of the grant of the patent: **14.08.2024**
(21) Application number: 16756485.5
(22) Date of filing: 26.02.2016
(51) Int. Cl.: A61K 9/22, A61K 9/20, A61K 9/24, A61K 31/137, A61K 31/4458, A61P 25/14

(54) **TRIPULSE RELEASE STIMULANT FORMULATIONS**
TRIPULSE-FREISETZENDE STIMULANSFORMULIERUNGEN
FORMULATIONS DE STIMULANT DE LIBÉRATION À TROIS IMPULSIONS

(30) Priority: 27.02.2015 US 201562121537 P
(43) Date of publication of application: 03.01.2018
(73) Proprietor: Cingulate Therapeutics LLC, Morristown, NJ 07960 (US)
(72) Inventor: BRAMS, Matthew, Morristown, NJ 07960 (US); SILVA, Raul, Morriston, NJ 07960 (US); STRAUGHN, Arthur, Morristown, NJ 07960 (US)
(74) Representative: Schiweck Weinzierl Koch Patentanwälte Partnerschaft mbB
(86) International application number: PCT/US2016/019877
(87) International publication number: WO 2016/138440

(56) References cited:
- US-A1- 2003 170 181
- US-A1- 2010 104 621
- US-B1- 6 217 904
- US-B1- 6 217 904
- US-B1- 6 322 819

## Description

### BACKGROUND OF THE INVENTION

Patients treated with stimulants for various disorders such as Attention Deficit Hyperactivity Disorder (ADHD) often take one or more doses during the patient's awake hours to achieve the therapeutic levels of stimulant needed. In one example, patients take a single dosage form that releases the stimulant in two discrete doses or pulses (e.g., Concerta^{®}; Adderall XR^{®}), with the second pulse coming about 3-5 hours after the first release. While usually providing therapeutically effective levels of stimulant for the first 8 hours or more after the initial administration, many patients require an additional dose to maintain the same therapeutic effects throughout an entire day. These patients often take a separate immediate release dosage form of the same drug later in the day to extend the therapeutic effect. Efforts to date that achieve this effect in a single dosage form have fallen short. Thus, there remains a need for a single, once-daily dosage form that delivers a stimulant drug in a controlled, precise and effective pulsatile drug delivery system that provides a therapeutic amount of stimulant throughout a patient's waking hours. US 2003/0170181 A1 relates to a method for treating a patient with a methylphenidate-responsive condition that is at a risk of abusing or becoming addicted to methylphenidate. The patient is first evaluated for an elevated risk of drug abuse or addiction through psychological evaluations and then treated with a methylphenidate product that includes an emesis-inducing agent that is inert when ingested orally and only produces emesis when snorted or taken intravenously or a topical analgesic that is inert when ingested orally and only produces irritation when snorted or taken intravenously. The method includes delivering the methylphenidate in a pulsatile delivery system such that the emesis-inducing agent or the topical analgesic is included in one of the pulsatile dosages. US 6,217,904 relates to pharmaceutical dosage forms provided for pulsatile delivery of d-threo-methylphenidate and a second CNS stimulant, i.e., release encapsulated drug in spaced apart "pulses." The second CNS stimulant may be an analeptic agent or a psychostimulant, with analeptic agents preferred. The dosage forms may comprise capsules housing compressed tablets or drug-containing beads or particles, or may comprise a tablet with the first, second and optionally third dosage units each representing an integral and discrete segment thereof. Methods of treatment using the pharmaceutical dosage forms are provided as well.

### SUMMARY OF THE INVENTION

The present invention relates to a pulsatile release dosage form for oral administration of a stimulant, comprising
(a) a first dosage unit comprising a first stimulant dose that is released substantially immediately following oral administration of the dosage form to a patient;
(b) a second dosage unit comprising a second stimulant dose, wherein the second stimulant dose is delayed until about 2 to about 5 hours following administration of the dosage form to a patient; and
(c) a third dosage unit comprising a third stimulant dose, wherein the third stimulant dose is delayed until about 5 to about 9 hours following oral administration of the dosage form to a patient;

wherein the dosage form is a tablet, and wherein the dosage units are incorporated at specific and controlled positions within the finished dosage form,
wherein the first, second and third dosage units each comprise a core that is at least partially surrounded by an erosion matrix,
wherein the core of the first dosage unit is partially surrounded by an erosion matrix at an edge of the first dosage unit such that at least one outer edge of the first dosage unit is not surrounded by the erosion matrix and the cores of the second and third dosage units are fully surrounded by the erosion matrix,
and wherein the erosion matrix comprises one or more of a swelling agent, lubricant, disintegrant, binder, and a pharmaceutically acceptable excipient; and
wherein the stimulant is methylphenidate, or a pharmaceutically acceptable salt thereof.

In some embodiments, the first dosage unit releases about 30% to about 40% of the total stimulant in the dosage form.

In some embodiments, the first dosage unit releases about 35% to about 40% of the total stimulant in the dosage form.

In some embodiments, the second dosage unit releases about 40% to about 50% of the total stimulant in the dosage form.

In some embodiments, the second dosage unit releases about 45% to about 50% of the total stimulant in the dosage form.

In some embodiments, the third dosage unit releases about 10% to about 20% of the total stimulant in the dosage form.

In some embodiments, the third dosage unit releases about 15% to about 20% of the total stimulant in the dosage form.

The present invention also relates to a once-daily dosage form of the invention for use in treatment of a disorder, condition or disease for which a stimulant is generally indicated.

In some embodiments, the disorder, condition or disease is ADHD.

In some embodiments,
a) the first stimulant dose is released substantially immediately in less than 10 minutes following oral administration of the dosage form to a patient and/or
b) the second stimulant dose is delayed until about 2 to about 5 hours following oral administration of the dosage form to a patient, and wherein the second stimulant dose is released over a time period of about 45 to 90 minutes and/or
c) the third stimulant dose is delayed until about 6 to about 8 hours following oral administration of the dosage form to a patient.

In some embodiments, the second stimulant dose is delayed until about 3 to about 4 hours following oral administration of the dosage form to a patient.

In some embodiments, the second stimulant dose is released over a time period of from about 60 minutes to about 90 minutes.

The claimed invention is defined in the appended claims.

A single dosage form that delivers a tripulse (i.e., three pulses) release profile is provided. According to the claimed invention, the dosage form includes at least three dosage units inside the final dosage form. By providing a single dosage form having multiple dosage units, the dosing frequency is decreased since precise blood levels of the active over a prolonged time period are controlled and achieved.

The dosage form as provided herein provides a pharmaceutically acceptable amount of active ingredient initially to the patient to cause the desired pharmacological response via a first dosage unit and delivers the remaining pharmaceutically acceptable amounts of active via a second and third dosage unit to maintain the pharmacological activity for a period of time in excess of the time expected from a single, immediate release dose and a second, delayed release dose.

According to one embodiment, the dosage form is a tablet that includes three dosage units. According to one embodiment, the tablet has an overall length of from about 10.00 mm to about 20.00 mm, or from about 13.00 mm to about 18.00 mm, or from about 13.00 mm to about 14.00 mm. According to one embodiment, the tablet has an overall length of from about 13.00 mm to about 14.00 mm.

According to another embodiment, the tablet has an overall width of about 6.00 mm to about 11.00 mm, or from about 6.00 mm to about 10.00 mm, or from about 6.00 mm to about 7.00 mm. According to another embodiment, the tablet has an overall width of about 6.00 mm to about 7.00 mm.

According to one embodiment, the tablet has an overall thickness of about 5.00 mm to about 10.00 mm, or from about 7.00 mm to about 9.00 mm, or from about 8.00 mm to about 9.00 mm.

According to the claimed invention, the first dosage unit includes a first stimulant dose that is released substantially immediately following oral administration of the dosage form to a patient (i.e. immediate release or IR). According to one embodiment, the first dosage unit reaches about 85% dissolution in less than about 20 minutes. According to another embodiment, the first dosage unit reaches 100% dissolution in less than 10 minutes. According to another embodiment, the first dosage unit reaches 100% in less than 5 minutes.

In certain embodiments, the first stimulant dose is from about 1 mg - 200 mg, or from about 2 mg - 100 mg, or from about 3 mg to about 60 mg. According to one embodiment, the first stimulant dose is from about 5 mg - 40 mg. According to another embodiment, the first stimulant dose is from about 10 mg - 30 mg.

According to one embodiment, the first dosage unit releases from about 30% to about 50% of the total stimulant in the dosage form, or from about 30% to about 40%, or from about 35% to about 40%.

According to the claimed invention, the second dosage unit includes a second stimulant dose that is delayed in release until about 2 hours to about 5 hours following administration of the dosage form to a patient (i.e. delayed release or DR). According to another embodiment, the second stimulant dose is delayed until about 3 to about 4 hours following administration of the dosage form to a patient. According to another embodiment, the second stimulant dose is delayed until about 4 hours following administration of the dosage form to a patient.

In certain embodiments, the second stimulant dose is from about 1 mg - 200 mg, or from about 2 mg - 100 mg, or from about 3 mg to about 60 mg. According to one embodiment, the second stimulant dose is from about 5 mg - 40 mg. According to another embodiment, the second stimulant dose is from about 10 mg - 30 mg.

According to one embodiment, the second dosage unit releases from about 35% to about 55% of the total stimulant in the dosage form, or from about 40% to about 50%, or from about 45% to about 50%.

According to one embodiment, the second dose is released over a time period of from about 30 minutes to about 100 minutes (i.e. sustained release or SR). According to another embodiment, the second dose is released over a time period of from about 45 minutes to about 90 minutes. According to another embodiment, the second dose is released over a time period of from about 60 minutes to about 90 minutes. According to another embodiment, the second dose is released over a time period of about 90 minutes.

According to the claimed invention, the third dosage unit includes a third stimulant dose that is delayed in release until about 5 hours to about 9 hours following oral administration of the dosage form to a patient (i.e. also delayed release or DR). According to one embodiment, the third stimulant dose is delayed until about 6 hours to about 8 hours following oral administration of the dosage form to a patient. According to another embodiment, the third stimulant dose is delayed until about 7 hours following oral administration of the dosage form to a patient.

In some embodiments, the third stimulant dose is from about 1 mg - 200 mg, or from about 2 mg - 100 mg, or from about 3 mg to about 60 mg. According to one embodiment, the third stimulant dose is from about 1 mg - 25 mg. According to one embodiment, the third stimulant dose is from about 2 mg - 20 mg, or from about 5 mg - 15 mg, or from about 8 mg - 12 mg.

According to one embodiment, the third dosage unit releases from about 5% to about 20% of the total stimulant in the dosage form, or from about 10% to about 20%, or from about 15% to about 20%.

The first, second and third dosage units are incorporated at specific and controlled positions within the finished dosage form. According to the claimed invention, each of these dosage units includes a core that is at least partially surrounded by an erosion matrix. The erosion matrix forms one or more outer layers or erosion barrier layers surrounding at least one edge of each of the cores. Two of the cores belonging to the second and third dosage units are entirely surrounded by the erosion matrix such that the cores are not immediately exposed to the dissolution media following oral administration of the dosage form to a patient. As for the first dosage unit, it is partially surrounded by the erosion matrix, for example, at an edge of the first dosage unit. In another embodiment, the core facilitating the immediate release of the first stimulant dose is substantially exposed to the dissolution media without being encompassed by the erosion matrix, but the cores of the second and third dosage units remain fully encompassed by the erosion matrix. The thickness or coating weight of each of the one or more outer layers of the erosion matrix may be adjusted to match the desired release profile for the dosage unit. The erosion matrix may include one or more of a swelling agent, lubricant, disintegrant, binder, and other pharmaceutically acceptable excipients.

The dosage forms as provided herein are to be administered orally in a pharmaceutically effective amount to a patient once daily to treat or prevent a variety of disorders, conditions and diseases. According to one embodiment, administration of at least one stimulant may be carried out in order to treat any disorder, condition or disease for which a stimulant is generally indicated. Such disorders, conditions and diseases include, for example, ADHD, narcolepsy and acute depression. Stimulants may also be used in the treatment of individuals suffering from cognitive decline associated with AIDS or AIDS-related conditions, mood elevation in terminally ill patients suffering from a disease such as cancer, binge eating disorder, chronic fatigue, menopausal executive function, sluggish cognitive tempo (SCT), autistic spectrum disorder, depression, conduct disorders, chemotherapy-induced lethargy and fibromyalgia.

The stimulant delivered according to the provided dosage form is methylphenidate or a pharmaceutically acceptable salt thereof.

### DESCRIPTION OF THE DRAWINGS

FIG. 1A provides a perspective view of one embodiment of a dosage form 100 in an almond-shaped tablet formulation, showing the three dosage units 102, 104 and 106.
FIG. 1B provides a top cross-sectional view of the dosage form 100 in an almond-shaped tablet formulation according to one embodiment, with measurements quoted in millimeter (mm).
FIG. 2 provides a plasma concentration time curve for a dosage form containing a 25 mg dose of d-methylphenidate according to one embodiment.
FIG. 3 provides a plasma concentration time curve for a dosage form containing a 25 mg dose of d-methylphenidate according to one embodiment.
FIG. 4 provides a plasma concentration time curve for a dosage form containing a 25 mg dose of d-methylphenidate according to one embodiment.

### DETAILED DESCRIPTION OF THE INVENTION

As used in the specification and the appended claims, the singular forms "a," "an" and "the" include plural referents unless the context clearly dictates otherwise.

"Active ingredient" and "pharmacologically active ingredient" are used herein to refer to a chemical material or compound which induces a desired pharmacologic effect.

"Stimulant" as used herein refers to any active pharmaceutical ingredient administered to induce temporary improvements in either mental or physical functions or both.

"Methylphenidate" as used herein, includes all four optical isomers of the compound (d-threo-methylphenidate, l-threo-methylphenidate, d-erythro-methylphenidate, and l-erythro-methylphenidate) and all pharmaceutically acceptable salts, including a racemic mixture of d-threo methylphenidate and l-threo methylphenidate as well as methylphenidate hydrochloride.

"Amphetamine" as used herein, includes optically pure d-form, its optically pure l-form, its optically pure dl-form, racemic mixtures of its d-, l-, and dl-forms, amphetamine phosphate, amphetamine succinate, methamphetamine, benzphetamine, phentermine, diethylpropion, phenmetrazine, phendimetrazine, chlorphentermine, clortermine, mazindol, phenylpropanolamine, dextroamphetamine, levoamphetamine, and lisdexamfetamine, as well as all pharmaceutically acceptable metabolites and prodrugs thereof.

"Pulsatile drug delivery system" or "Pulsed-release drug delivery system" refers to a typically single dosage form that aims to release one or more drugs in multiple pulses in a programmed pattern, i.e. at appropriate, predetermined times that are separated by release-free intervals or lag-time and in appropriate, predetermined amounts.

"Effective amount" or "pharmaceutically effective amount" of an agent as provided herein is meant to refer to a nontoxic but sufficient amount of an active ingredient to provide the desired therapeutic effect.

It is to be noted that terms such as "first", "second", "third", "top", "bottom", "upper", "lower" are applied foremostly for purposes of clarity and distinguishing one element or object from another. Such terms, especially "top", "bottom", "upper" and "lower" are relative and dependent of the orientation of an object (e.g. tablet) which may be same as or different from the orientations of the same object presented in the figures provided herein.

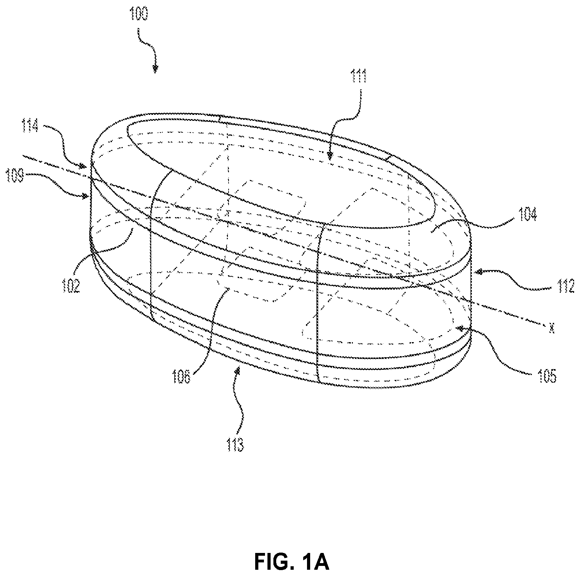

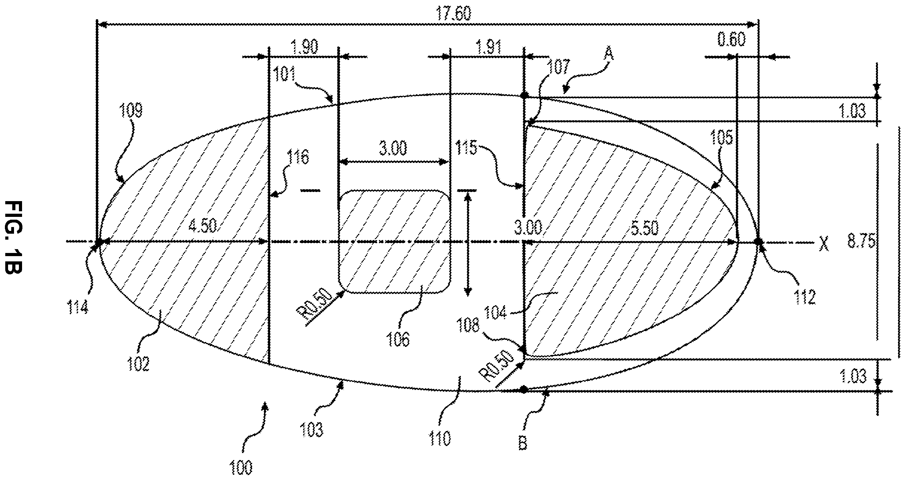

FIGS. 1A and 1B respectively provide a perspective view and a top cross-sectional view of an embodiment of a dosage form 100 in an almond-shaped tablet formulation. The dosage form 100 is composed of a top flat surface 111, a bottom flat surface 113 and side convex surfaces 101 and 103. The side convex surfaces 101 and 103 each have a peak point, A and B, respectively. The distance between the peak points A and B, which define the overall width of the dosage form 100, is from about 6.00 mm to about 11.00 mm, or from about 6.00 mm to about 10.00 mm, or from about 6.00 mm to about 7.00 mm. In one embodiment, as shown in FIG. 1B, the distance between the peak points A and B is about 8.75 mm.

The two side convex surfaces (101, 103) meet and join at two ends, namely broad end 112 and narrow end 114, which define the length of the dosage form 100 along the longitudinal axis x. The distance between the broad end 112 and the narrow end 114, which is the overall length of the dosage form 100, is from about 10.00 mm to about 20.00 mm, or from about 13.00 mm to about 18.00 mm, or from about 13.00 mm to about 14.00 mm. In one embodiment, for example the one shown in FIG. 1B, the dosage form 100 has a length of about 17.60 mm.

With respect to the overall thickness of the dosage form 100, which is measured from the top flat surface 111 to the bottom flat surface 113, the range is from about 5.00 mm to about 10.00 mm, or from about 7.00 mm to about 9.00 mm, or from about 8.00 mm to about 9.00 mm. In one embodiment, the dosage form 100 has a thickness of 8.75 mm.

As illustrated in FIGS. 1A and 1B, each of the first, second and third dosage units (102, 104, and 106, respectively) is compressed and located separate and apart from one another within the dosage form 100. An erosion matrix 110 makes up the rest of the dosage form 100 that is not occupied by the three dosage units 102, 104 and 106.

The first dosage unit 102 is designed for release of a first stimulant dose substantially immediately following oral administration of the dosage form 100 to a patient (i.e. immediate release or IR). Accordingly, the first dosage unit 102 is located proximal to the narrow end 114 and is shaped in a manner (e.g. the dome shape of FIGS. 1A and 1B, but not so limited) such that at least one outer edge 109 of the first dosage unit 102 is not surrounded by the erosion matrix 110 and is exposed to the dissolution media to facilitate immediate release of the first dosage unit 102. The first dosage unit 102 is, however, surrounded by the erosion matrix 110 at both the top and bottom flat surfaces (111, 113) and inner edge 116 so that the first dosage unit 102 can be held together with the rest of the dosage form 100.

The first dosage unit 102 is partially surrounded by an erosion matrix 110 according to the appended claims and partially exposed to the dissolution media.

The first dosage unit 102 has a length (along the longitudinal axis x) of about 2.00 mm to 6.00 mm, or from about 3.00 mm to about 6.00 mm, or from about 4.00 mm to about 5.00 mm. In the embodiment shown in FIG. 1B, the first dosage unit 102 has a length of about 4.50 mm.

The thickness of the first dosage unit 102 is the same as the overall thickness of the dosage form 100, which is in the range of from about 6.00 mm to about 14.00 mm, or from about 6.00 mm to about 13.00 mm, or from about 6.00 mm to about 11.00 mm, or from about 7.00 mm to about 10.00 mm, or from about 8.00 mm to about 9.00 mm or about 8.50 mm to about 9.00 mm. In one embodiment, the first dosage unit 102 has a thickness of 8.75 mm.

According to one embodiment, the first dosage unit 102 releases from about 30% to about 50% of the total stimulant in the dosage form, or from about 30% to about 40%, or from about 35% to about 40%.

The second dosage unit 104 is designed for release of a second stimulant dose that is delayed until about 2 hours to about 5 hours following administration of the dosage form to a patient (i.e. delayed release or DR). According to one embodiment, the second stimulant dose is delayed until about 3 to about 4 hours following administration of the dosage form to a patient. According to one embodiment, the second stimulant dose delayed until about 4 hours following administration of the dosage form to a patient.

According to one embodiment, the second dosage unit 104 releases from about 35% to about 55% of the total stimulant in the dosage form, or from about 40% to about 50%, or from about 45% to about 50%.

The second dosage unit 104, located proximal to the broad end 112, is shaped in a manner (e.g. dome-shaped, arrowhead-shaped but not so limited) such that one leading edge 105, which is unexposed to the dissolution media and shielded by the erosion matrix 110, begins erosion at a delayed point after ingestion. The distance or gap between the broad end 112 and the leading edge 105 is about 0.25 mm to about 1.00 mm, or from about 0.40 mm to about 0.80 about, or from about 0.45 mm to about 0.75 mm, or from about 0.50 mm to about 0.70 mm, or from about 0.50 mm to about 0.60 mm. In one embodiment, such as the one shown in FIG. 1B, the gap between the broad end 112 and the leading edge 105 is about 0.50 mm.

The second dosage unit 104 has a length (along longitudinal axis x) of about 2.50 mm to about 7.50 mm, or from about 3.00 mm to about 6.00 mm, or from about 4.00 mm to about 6.00 mm, or from about 5.00 mm to about 6.00 mm, or from about 5.50 mm to about 6.00 mm or about 5.00 mm to about 5.50 mm. As shown in FIG. 1B, the second dosage unit 104 has a length of about 5.50 mm.

In order to facilitate the delayed and sustained release of the second dosage unit 104, it is particularly advantageous that, in addition to the leading edge 105, the second dosage unit 104 in its entirety, including side corners 107, 108 and inner edge 115, is unexposed to the dissolution media and shielded by the erosion matrix 110, as most clearly depicted in FIG. 1B. The distance between side corner 107 and point A and the distance between side corner 108 and point B are advantageously equal but are not so limited, measuring at about 0.25 mm to about 3.00 mm, or about 0.50 mm to about 2.50 mm, or about 0.75 to about 2.25 mm, or about 1.00 mm to about 2.00 mm or about 1.00 mm to about 1.50 mm or about 1.50 mm to about 2.00 mm. In the embodiment of FIG. 1B, the distance between side corner 107 and point A and the distance between side corner 108 and point B are equal at 1.03 mm.

Further to the delayed release of the second dose by the second dosage unit 104, the second dose is released over a time period of from about 30 minute to about 100 minutes (i.e. sustained release or SR). According to another embodiment, the second dose is released over a time period of from about 45 minutes to about 90 minutes. According to another embodiment, the second dose is released over a time period of from about 60 minutes to about 90 minutes. According to another embodiment, the second dose is released over a time period of about 90 minutes.

Still referring to FIGS. 1A and 1B, the third dosage unit 106 is substantially rectangular-shaped and located in a vertically and horizontally centered portion of the dosage form 100, unexposed to the dissolution media and shielded by the erosion matrix 110, such that the third dosage unit 106 begins erosion at a point in time after complete release of the first dosage unit 102 and the second dosage unit 104. Accordingly, the third dosage unit 106 is disposed between the first and second dosage units 102, 104 in the middle of the dosage form 100. According to a particular embodiment, dimensions of the rectangular-shaped third dosage unit 106 are about 2.00-5.00 mm × about 2.00-5.00 mm (length along longitudinal axis *x* × width perpendicular to the longitudinal axis x), or about 2.50-4.50 mm × about 2.50-4.50 mm, or about 2.50-3.50 mm × about 2.50-3.50 mm or about 3.00-4.00 mm × about 3.00-4.00 mm. In one embodiment, the rectangular shape of the third dosage unit 106 has a dimension of about 3.00 mm × about 3.00 mm.

The third dosage unit 106 has a thickness of from about 1.00 mm to about 8.00 mm, or from about 2.50 mm to about 7.50 mm, or from about 3.00 mm to about 6.00 mm, or from about 4.00 to about 5.00 mm. In one embodiment, the third dosage unit 106 has a thickness of about 5.00 mm.

According to the claimed invention, the third dosage unit includes a third stimulant dose that is delayed in release until about 5 hours to about 9 hours following oral administration of the dosage form to a patient (i.e. also delayed release or DR). According to one embodiment, the third stimulant dose is delayed until about 6 hours to about 8 hours following oral administration of the dosage form to a patient. According to another embodiment, the third stimulant dose is delayed until about 7 hours following oral administration of the dosage form to a patient.

In some embodiments, the third stimulant dose is from about 1 mg - 200 mg, or from about 2 mg - 100 mg, or from about 3 mg to about 60 mg. According to one embodiment, the third stimulant dose is from about 1 mg - 25 mg. According to one embodiment, the third stimulant dose is from about 2 mg - 20 mg, or from about 5 mg - 15 mg, or from about 8 mg - 12 mg.

According to one embodiment, the third dosage unit releases from about 5% to about 20% of the total stimulant in the dosage form, or from about 10% to about 20%, or from about 15% to about 20%.

The distance between the first and third dosage units (102, 106) is from about 1.50 mm to about 2.50 mm, or from about 1.50 mm to about 2.00 mm, or from about 1.75 mm to about 2.00 mm, or from about 1.80 mm to about 2.00 mm, or from about 1.90 mm to about 1.95 mm. As shown in FIG. 1B, the distance between the first and third dosage units (102, 106) is about 1.90 mm.

Similarly, the distance between the second and third dosage units (104, 106) is about 1.50 mm to about 2.50 mm, or from about 1.50 mm to about 2.00 mm, or from about 1.75 mm to about 2.00 mm, or from about 1.80 mm to about 2.00 mm, or from about 1.90 mm to about 1.95 mm. As shown in FIG. 1B, the distance between the second and third dosage units (104, 106) is about 1.91 mm.

Referring to FIG. 1B, it is shown that the second and third dosage units 104 and 106 have rounded edges where the border radius of each corner, including side corners 107, 108 of the second dosage unit 104, is from about 0.25 mm to about 0.60 mm, or from about 0.40 to about 0.60 mm, or about 0.50 mm.

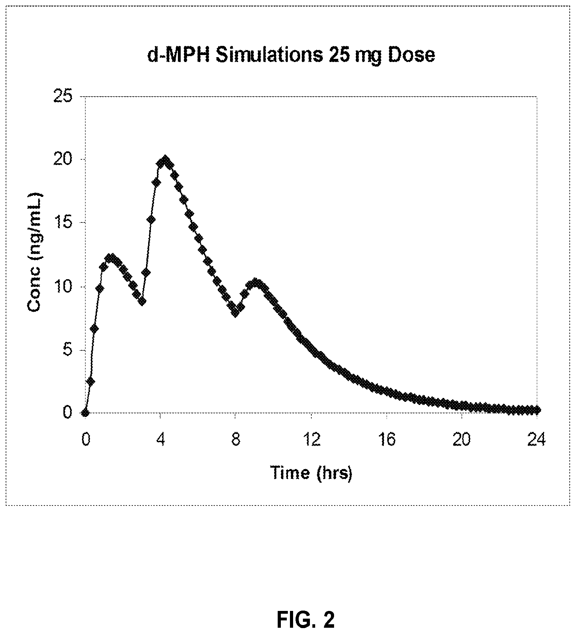

FIGS. 2-4 illustrate a plasma concentration time curve according to three dosage form embodiments of 25 mg of d-methylphenidate. Simulations of d-methylphenidate plasma concentrations were accomplished utilizing a numerical integration software package (STELLA) which allows one to convolute *in vitro* drug dissolution data to *in vivo* drug concentrations. These simulations are based on a specified percent of drug being released at different times from a triple pulse dosage form in the gastrointestinal tract. Upon release, first order absorption into the blood and elimination from the body follows predictions of a one-compartment open pharmacokinetic model.

In accordance with the present disclosure, the first dosage unit releases from about 30% to about 50% of the total stimulant in the dosage form, or from about 30% to about 40%, or from about 35% to about 40%. The second dosage unit releases from about 35% to about 55% of the total stimulant in the dosage form, or from about 40% to about 50%, or from about 45% to about 50%. The third dosage unit releases from about 5% to about 20% of the total stimulant in the dosage form, or from about 10% to about 20%, or from about 15% to about 20%.

The profile of the dosage form giving rise to the simulated curve of FIG. 2 is as follows:

**Table 1. Dosage form profile.**

| Profile Description | Parameter (%) |
|---|---|
| First Dosage Unit | 40 |
| Second Dosage Unit | 45 |
| Third Dosage Unit | 15 |
| % Total | 100 |
| Delay DR1 (hrs) | 3.0 |
| Delay DR2 (hrs) | 8.0 |

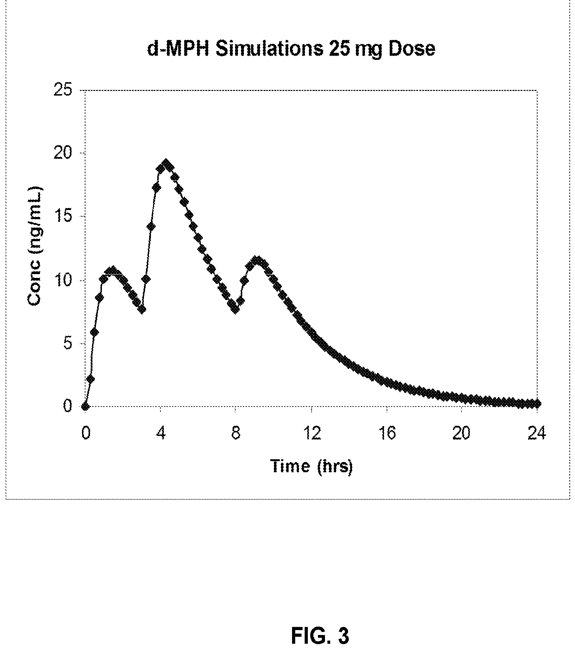

The profile of the dosage form giving rise to the simulated curve of FIG. 3 is as follows:

**Table 2. Dosage form profile.**

| Profile Description | Parameter (%) |
|---|---|
| First Dosage Unit | 35 |
| Second Dosage Unit | 45 |
| Third Dosage Unit | 20 |
| % Total | 100 |
| Delay DR1 (hrs) | 3.0 |
| Delay DR2 (hrs) | 8.0 |

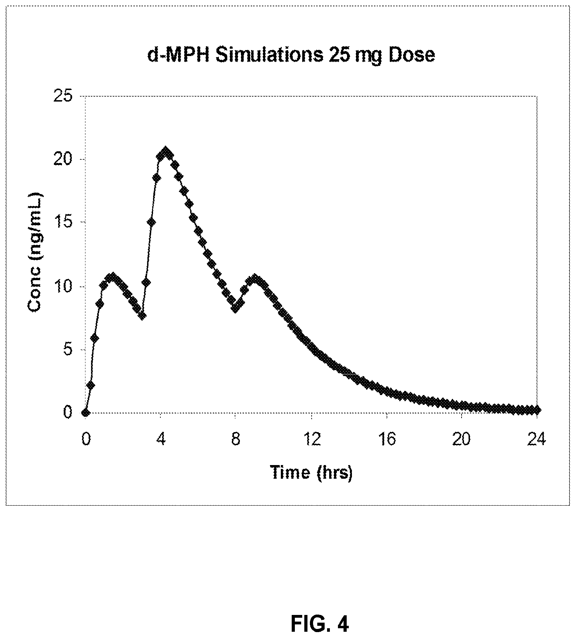

The profile of the dosage form giving rise to the simulated curve of FIG. 4 is as follows:

**Table 3. Dosage form profile.**

| Profile Description | Parameter (%) |
|---|---|
| First Dosage Unit | 35 |
| Second Dosage Unit | 50 |
| Third Dosage Unit | 15 |
| % Total | 100 |
| Delay DR1 (hrs) | 3.0 |
| Delay DR2 (hrs) | 8.0 |

As seen in FIGS. 2-4, the tripulse release profile of the dosage form of the present invention, allows for a prolonged duration of stimulant action with improved tolerability compared to dual-release dosage forms such as Concerta^{®}, Adderall XR^{®} and/or multiple, daily administration of immediate release (IR) stimulant products.

Additionally, the tripulse release profile of the dosage form creates a controlled decline of the plasma concentration of the stimulant. The controlled decline is advantageous in that it reduces or minimizes crash episodes or withdrawal symptoms that are commonly associated with the dual-release Concerta^{®} and Adderall XR^{®}. Those who are on Concerta^{®} prescription frequently complain about such unpleasant crash episodes at about 5 to 7 hours after taking the medication, where the common symptoms include nausea, digestive discomfort, depressive feelings, lack of energy, irritability, reduced ability to focus, increased anxiety, etc. Such crash episodes can be dangerous and present medical emergencies.

The shape of the dosage units may be modified such that various geometrical shapes may be strategically located within the dosage unit. Suitable dosage unit shapes include, but are not limited to, round, triangular, square, rectangular, almond-shaped, oval-shaped, capsule-shaped, pillow-shaped star-shaped and teardrop shaped. According to a particular embodiment, at least one dosage unit as provided herein has a surface substantially exposed to an exterior surface of the dosage form (see FIG. 1B). By placing a dosage unit close to an edge of the dosage form, the release profile is altered such that the dosage units begin erosion quicker upon ingestion. For example, at least one surface of the first dosage unit may be exposed to the exterior of the dosage form such that the first dosage unit begins releasing substantially immediately upon ingestion.

The dosage forms disclosed herein are suitable for the delivery of an effective amount of at least one stimulant and salts thereof. At least one other active ingredient may be combined with the stimulant in a single dosage unit within the dosage form, or one or more dosage units within the dosage form may comprise the additional active ingredient. Salts of the active ingredients used in conjunction with the present dosage forms may be obtained commercially or can be prepared using standard procedures known to those skilled in the art of synthetic organic chemistry. Suitable stimulants include, but are not limited to: amphetamine, dextroamphetamine, amphetaminil, bemegride, benzphetamine, benzphetamine hydrochloride, brucine, chlorphentermine, clofenciclan, clortermine, deanol acetamidobenzoate, demanyl phosphate, dexoxadrol, diethpropion, doxapram hydrochloride, N-ethylamphetamine, ethamivan, etifelmin, etryptamine, fencamfamine, fenethylline, fenosolone, fenfluramine, flurothyl, hexacyclonate sodium, homocamfin, mazindol, megexamide, methamphetamine, nicotinic agonists, nikethamide, pentylenetetrazole, phenidimetrazine, phendimetrazine tartrate, phenmetrazine, phenmetrazine hydrochloride, phentermine, picrotoxin, pipradrol, pipradrol hydrochloride, prolintane, pyrovalerone, racephedrine, racephedrine hydrochloride, and tetrahydrobenzothienopyridines. According to a particular embodiment, the stimulant is dexmethylphenidate, dextroamphetamine, or a combination thereof. The stimulants provided herein may be in the form of a pharmaceutically acceptable salt, prodrug or other derivative or active metabolite.

Optional components present in the dosage form include, but are not limited to, additional binders, lubricants, disintegrants, stabilizers, surfactants, coloring agents, and diluents. Suitable diluents include, but are not limited to, dicalcium phosphate dihydrate, calcium sulfate, lactose, cellulose, kaolin, mannitol, sodium chloride, dry starch, hydrolyzed starches, silicon dioxide, titanium oxide, alumina, talc, microcrystal-line cellulose, and powdered sugar. Suitable binder materials include, but are not limited to, starch (including corn starch and pregelatinized starch), gelatin, sugars (including sucrose, glucose, dextrose, lactose and sorbitol), polyethylene glycol, waxes, natural and synthetic gums, e.g., acacia, tragacanth, sodium alginate, polyvinylpyrrolidone, celluloses, and Veegum, and synthetic polymers such as polymethacrylates and polyvinylpyrrolidone. Suitable lubricants include, but are not limited to, magnesium stearate, calcium stearate, stearic acid, glyceryl behenate, and polyethylene glycol. Suitable disintegrants include, but are not limited to, starches, clays, celluloses, algins, gums or crosslinked polymers. Suitable surfactants include, but are not limited to, those containing carboxylate, sulfonate and sulfate ions, associated with cations such as sodium, potassium and ammonium ions; long alkyl chain sulfonates and alkyl aryl sulfonates such as sodium dodecylbenzene sulfonate; dialkyl sodium sulfosuccinates, such as sodium bis-(2-ethylhexyl)-sulfosuccinate; and alkyl sulfates such as sodium lauryl sulfate. Nontoxic auxiliary substances such as wetting or emulsifying agents, pH buffering agents, preservatives may also be included in the dosage form or in the individual drug-containing dosage units.

The dosage forms as provided herein include at least three dosage units. Each dosage unit as provided includes a core that contains a releasable stimulant dose. Each core may include up to 99.9% active ingredient. According to a particular embodiment, the core may include up to 50% active ingredient. According to one embodiment, the core size within each dosage unit can be manufactured to be substantially the same size by adjusting the active ingredient concentration within the core.

In some embodiments, each core contains, as a stimulant dose and in weight percentages based on the total weight of the core: 8.00-12.00% of dexmethylphenidate hydrochloride, 70.00-80.00% of mannitol (Pearlitol^{®} 310DC), 8.00-10.00% of croscarmellose sodium (Ac-Di-Sol, Type SD-711), 4.00-5.00% of Providone K-12 and 1.00-2.00% of magnesium stearate.

According to one particular embodiment, each core contains the following composition as a stimulant dose:

**Table 4, Core composition.**

| Ingredient | % w/w |
|---|---|
| Dexmethylphenidate hydrochloride | 10.00 |
| Mannitol (Pearlitol^{®} 310DC) | 74.72 |
| Croscarmellose sodium (Ac-Di-Sol, Type SD-711) | 9.52 |
| Providone K-12 | 4.76 |
| Magnesium stearate | 1.00 |
| Total | 100.00 |

The remainder of the dosage form includes an erosion matrix that at least partially surrounds the first and completely surrounds the second and third dosage units. The erosion matrix includes a blend of one or more disintegrant, binding agent, lubricating or release agent, dye/pigment, or other pharmaceutically acceptable excipient. Suitable binding agents include, for example, hydroxyethyl cellulose (HEC), hydroxypropyl cellulose (HPC), hydroxypropyl methylcellulose (HPMC), methyl cellulose, ethyl cellulose, cellulose ethers, cellulose esters, polyvinyl pyrrolidone, copovidone, polyvinyl alcohol, amino methylacrylate copolymer, acrylic polymers or a combination thereof. Suitable lubricating or release agents include glyceryl behenate, calcium stearate, and sodium stearate. The erosion matrix may further include one or more disintegrants such as, for example, microcrystalline cellulose, starches, clays, celluloses, algins, gums or crosslinked polymers (e.g., crospovidone, polivinylpyrrolidones)). The erosion matrix may further include other excipients such as, for example, lactose monohydrate and dibasic calcium phosphate. The erosion matrix may further include a pharmaceutically acceptable dye or pigment such that that each dosage unit is colored the same or different colored.

The erosion matrix may further include low molecular weight hypromellose which may be modified with a hydrophobic component which provides a barrier for protection of the stimulant leading to delayed release. Suitable hydrophobic components include any lipid-based lubricant suitable for direct compression such as, for example, Compritol ATO 888 (Glyceryl Behenate available from Gattefossé, France).

In certain embodiments, the erosion matrix or each of the one or more outer layers forming the erosion matrix is made up of, in weight percentages based on the total weight of the matrix: 80.00-90.00% of hypromellose (Methocel E-5) USP, 8.00-10.00% of glyceryl behenate (Compritol 888) NF, 1.0-1.5% of colloidal silicon dioxide (Aerosil 200 Pharma) USP/EP (intra-granular) and 2.0-2.5% of colloidal silicon dioxide (Aerosil 200 Pharma) USP/EP (extra-granular).

According to a particular embodiment, the erosion matrix or each of the one or more outer layers forming the erosion matrix has the following composition:

**Table 5. Erosion matrix composition.**

| Ingredient | % w/w |
|---|---|
| Hypromellose (Methocel E-5) USP | 87.2 |
| Glyceryl behenate (Compritol 888) NF | 9.78 |
| Colloidal silicon dioxide (Aerosil 200 Pharma) USP/EP (Intra-granular) | 1.0 |
| Colloidal silicon dioxide (Aerosil 200 Pharma) USP/EP (Extra-granular) | 2.0 |
| Total | 100.00 |

In alternative embodiments, the erosion matrix may comprise a wax and a low-substituted hydroxypropyl cellulose (L-HPC) such as LH-32, LH-11 and LH-21 as set forth in one or more of U.S. Patent Application Publication 2013/0022677 A1, U.S. Patent Application Publication 2013/0022676 A1 and U.S. Patent Application Publication 2013/0017262 A1.

In one of the alternative embodiments, the erosion matrix may comprise a wax and LH-32, which is a particular type of low substituted hydroxypropyl cellulose (L-HPC) and may be obtained from Shin-Etsu Chemical Co., Ltd., Tokyo, Japan. L-HPCs are insoluble in water and comprise a glucose backbone which is substituted to a minimal extent by hydroxypropyl groups. LH-32 is micronised, with a mean particle diameter of 20 µm. LH-32 has a molecular weight of 115,000 and a hydroxypropyl cellulose content of 8%. Examples of waxes include beeswax, carnuba wax, microcrystalline wax and hydrogenated castor oil. According to a particular embodiment, the wax is a glyceryl ester, such as glycerol behenate.

In other alternative embodiments, the L-HPC is LH-11 or LH-21. LH-11 and LH-21 are particular types of L-HPC and may be obtained from Shin-Etsu Chemical Co., Ltd., Tokyo, Japan. L-HPCs are insoluble in water and comprise a glucose backbone which is substituted to a minimal extent by hydroxypropyl groups. LH-11 is mostly fibrous and has a mean particle size of 55 µm. LH-11 has a hydroxypropyl content of around 11% and a molecular weight of around 130,000. LH-21 is moderately fibrous and has a mean particle size of 45 µm. LH-21 has a molecular weight of around 120,000 and a hydroxypropyl content of around 11%.

In one embodiment, the erosion matrix comprises a wax and LH-32/LH-21/LH-11 in a ratio of 20:80 to 50:50 w/w. In another embodiment, the erosion matrix comprises a wax and LH-32/LH-21/LH-11 in a ratio of 25:75 to 40:60 w/w. In yet another embodiment, the erosion matrix comprises a wax and LH-32 in a ratio of 25:75 to 35:65 w/w, or 30:70 w/w. In a further embodiment, the wax and LH-32/LH-21/LH-11 are present in a ratio of 45:55 to 55:45 w/w, or 50:50 w/w.

The dosage forms as provided may be manufactured in a single step according to dry coating manufacturing methods as set forth in U.S. Patent No. 7,811,488, U.S. Patent No. 7,132,072, U.S. Patent No. 7,713,455, U.S. Patent No. 7,581,941. The machinery utilized in such methods may produce each dosage form with dosage units via a compression molding process that utilizes a double punch structure. The double punch structure includes a central punch and an outer punch surrounding the outer periphery of the central punch. The central punch and the outer punch perform sliding motions as well as compressing operations.

Manufacturing steps for the dosage forms provided herein include an initial step of positioning the lower central punch with respect to the lower outer punch to create a space above the lower central punch and inside the lower outer punch. Next, a first molding material such as, for example, the erosion matrix, is supplied into the space above the lower central punch and inside the lower outer punch, and onto an upper surface of the lower outer punch from a feeding shoe. The first molding material may form the lower outer layer of the core. The lower central punch and the upper central punch are driven to compression mold the first molding material between the lower central punch and the upper central punch. The upper outer punch is spaced a certain distance from the lower outer punch so as to prevent contact between the upper outer punch and the lower outer punch and between the upper outer punch and the first molding material. Next, pressured air is discharged and/or suction is applied to remove any unmolded first molding material (e.g., lower outer surface of dosage form) residue on the lower outer punch while the molded first molding material is gripped and held in place between the lower central punch and the projected upper central punch.

Similar manufacturing steps are then repeated to form at least one core containing a stimulant as well as any further portions of the one or more outer layers of the erosion matrix surrounding at least one edge of the core. Particularly the steps may be repeated to form the inner portion of the dosage form where multiple cores may be located as well as the upper, outer top and sides of the core's one or more outer layers to form the dosage form. The configuration of the central punches may be adjusted or otherwise adapted to form multiple core layers of a variety of shapes as provided herein.

Additionally, the manufacturing process of the dosage forms provided herein includes a dry granulation process (i.e. formation of multi-particulate granules from adherence of powder particles to another in the absence of a granulation liquid). The powder blend is subjected to compaction by applying a force onto the powder, which in general causes a considerable size enlargement. In accordance with the present disclosure, the compaction is obtained using the roller compaction technology, i.e. with a roller compactor which is a separate apparatus from the tablet press, where the powder is squeezed between two counter-rotating rollers of the roller compactor to produce a continuous sheet or ribbon of materials. Through the dry granulation-roller compaction step as part of the manufacturing process, the flow of the erosion matrix is improved so that the required delayed before the release of the cores of the second and third dosage units can be maintained and once the release is initiated, all of the core content will be released.

In another embodiment, the manufacturing steps and processes of the dosage forms described herein may be carried out with an apparatus having a plurality of upper and lower ram pairs arranged around a rotatable mold table, i.e. a rotary pelleting machine, a rotary tablet press, a rotary compression molding machine as set forth in one or more of U.S. Patent No. 4,452,579, U.S. Patent No. 4,569,650, U.S. Patent No. 5,462,427, U.S. Patent No. 6,050,798, U.S. Patent No. 7,553,147, U.S. Patent No. 7,967,572 and U.S. Patent No. 8,167,598.

The thickness of each of the one or more outer layers constituting the erosion matrix for each core within a dosage unit may be adjusted by adjusting the fill weights for the lower and upper layers which form the outer layer. The thickness of an outer layer may be adjusted to achieve the desired delayed release profile for each dosage unit. The overall shape and dimensions of the dosage form and internal dosage units may be altered by changing the shape of the central punch.

According to a particular embodiment, the central punch and the outer punch operate in a manner such that the core and the one or more outer layers of the erosion matrix of the dosage unit are integrally molded. The machinery provided may place each dosage unit at a specified location within each dosage form. A variety of pulsatile drug delivery profiles may be achieved due, in part, to the placement or location of the dosage units.

The rate of dissolution of the dosage units within the patient's digest tract may be controlled chemically via the one or more outer layers and various excipients provided herein, as well as physically, through the extent of compression of such components in the core and one or more outer layers. According to a particular embodiment, a compression force of from about 0.1 kN to about 10 kN may be applied via the central punch to form the core. The compression force applied to the core may be adjusted such that an increase in compression force results in an increase in the time to when release is initiated.

### EXAMPLE 1

A pulsatile release dosage form such as those illustrated in FIGS. 1A and 1B may be manufactured with the apparatus and steps as provided herein. The process may utilize a compression, double-punch molding apparatus that includes an upper punch and a lower punch which are arranged in the vertical direction of a die. Each of the upper punch and the lower punch has a double structure (i.e., two-cam system) that includes a central punch and an outer punch surrounding the outer periphery of the central punch. The central punch and the outer punch carry out independent sliding motions as well as compressing operations.

To form a lower portion of an outer layer of the dosage form, the lower central punch may be positioned with respect to the lower outer punch to create a space above the lower central punch and inside the lower outer punch (lower center punch slides down with lower outer punch acting as a die). Next, a first erosion matrix material is supplied from a feeding shoe into the space above the lower central punch and inside the lower outer punch, and onto an upper surface of the lower outer punch. The lower central punch and the upper central punch may then be driven to compress mold the first erosion matrix to form a lower portion of an outer layer. Any erosion matrix material residue may then be removed by discharging pressured air to the area. The molded lower portion of the outer layer remains gripped and held in place between the lower central punch and the projected upper central punch.

Next, the core composition including the active ingredient may then be supplied on top of the formed lower portion. The central punches are then driven to compress the active ingredient to form multiple core pharmacological layers. The configuration of the central punch dictates the number of cores formed and the respective shape. Finally, the lower central and outer punches may then slide down creating space for the erosion matrix to enter for forming the remainder of the outer layer. A compression of the lower central punch pushes the core layers up into the unmolded erosion matrix and encases the core. A final compression is then performed by the upper and lower punches with both the central and outer punches clutched together (i.e., compression forms sides and top of outer layer).

As will be appreciated by those skilled in the art, the specific components listed in the above tables may be replaced with other functionally equivalent components, e.g., diluents, binders, lubricants, fillers, coatings, and the like.

While the present invention has been described in detail, the present invention is not limited to the foregoing embodiments described above. Modifications may be made without departing from the scope of the present invention. The claimed invention is defined in the appended claims.

## Claims

1. A pulsatile release dosage form for oral administration of a stimulant, comprising
(a) a first dosage unit comprising a first stimulant dose that is released substantially immediately following oral administration of the dosage form to a patient;
(b) a second dosage unit comprising a second stimulant dose, wherein the second stimulant dose is delayed until about 2 to about 5 hours following administration of the dosage form to a patient; and
(c) a third dosage unit comprising a third stimulant dose, wherein the third stimulant dose is delayed until about 5 to about 9 hours following oral administration of the dosage form to a patient;
wherein the dosage form is a tablet, and wherein the dosage units are incorporated at specific and controlled positions within the finished dosage form,
wherein the first, second and third dosage units each comprise a core that is at least partially surrounded by an erosion matrix,
wherein the core of the first dosage unit is partially surrounded by an erosion matrix at an edge of the first dosage unit such that at least one outer edge of the first dosage unit is not surrounded by the erosion matrix and the cores of the second and third dosage units are fully surrounded by the erosion matrix,
and wherein the erosion matrix comprises one or more of a swelling agent, lubricant, disintegrant, binder, and a pharmaceutically acceptable excipient; and
wherein the stimulant is methylphenidate, or a pharmaceutically acceptable salt thereof.

2. The dosage form of claim 1, wherein the first dosage unit releases about 30% to about 40% of the total stimulant in the dosage form.

3. The dosage form of claim 1, wherein the first dosage unit releases about 35% to about 40% of the total stimulant in the dosage form.

4. The dosage form of claim 1, wherein the second dosage unit releases about 40% to about 50% of the total stimulant in the dosage form.

5. The dosage form of claim 1, wherein the second dosage unit releases about 45% to about 50% of the total stimulant in the dosage form.

6. The dosage form of claim 1, wherein the third dosage unit releases about 10% to about 20% of the total stimulant in the dosage form.

7. The dosage form of claim 1, wherein the third dosage unit releases about 15% to about 20% of the total stimulant in the dosage form.

8. The once-daily dosage form of any one of claims 1-7 for use in treatment of a disorder, condition or disease for which a stimulant is generally indicated.

9. The once-daily dosage form for the use of claim 8, wherein the disorder, condition or disease is ADHD.

10. The pulsatile release dosage form according to any one of claim 1, wherein
a) the first stimulant dose is released substantially immediately in less than 10 minutes following oral administration of the dosage form to a patient and/or
b) the second stimulant dose is delayed until about 2 to about 5 hours following oral administration of the dosage form to a patient, and wherein the second stimulant dose is released over a time period of about 45 to 90 minutes and/or
c) the third stimulant dose is delayed until about 6 to about 8 hours following oral administration of the dosage form to a patient.

11. The dosage form of claim 10, wherein the second stimulant dose is delayed until about 3 to about 4 hours following oral administration of the dosage form to a patient.

12. The dosage form of claim 10, wherein the second stimulant dose is released over a time period of from about 60 minutes to about 90 minutes.

## Patentansprüche

1. Dosierungsform mit pulsatibler Freisetzung zur oralen Verabreichung eines Stimulans, umfassend
(a) eine erste Dosierungseinheit, die eine erste Stimulansdosis umfasst, die im Wesentlichen unmittelbar nach der oralen Verabreichung der Darreichungsform an einen Patienten freigesetzt wird;
(b) eine zweite Dosierungseinheit, die eine zweite Stimulansdosis umfasst, wobei die zweite Stimulansdosis bis etwa 2 bis etwa 5 Stunden nach Verabreichung der Darreichungsform an einen Patienten verzögert wird; und
c) eine dritte Dosierungseinheit, die eine dritte Stimulansdosis umfasst, wobei die dritte Stimulansdosis bis etwa 5 bis etwa 9 Stunden nach der oralen Verabreichung der Darreichungsform an einen Patienten verzögert wird; wobei die Darreichungsform eine Tablette ist, und wobei die Dosierungseinheiten an bestimmten und kontrollierten Positionen innerhalb der fertigen Darreichungsform eingearbeitet sind, wobei die erste, zweite und dritte Darreichungseinheit jeweils einen Kern umfassen, der zumindest teilweise von einer Darreichungsform nach Anspruch 1, wobei die erste Dosierungseinheit etwa 35 % bis etwa 40 % des gesamten Stimulans in der Darreichungsform freisetzt.
wobei der Kern der ersten Dosiereinheit an einem Rand der ersten Dosiereinheit teilweise von einer Erosionsmatrix umgeben ist, so dass mindestens ein äußerer Rand der ersten Dosiereinheit nicht von der Erosionsmatrix umgeben ist und die Kerne der zweiten und dritten Dosiereinheit vollständig von der Erosionsmatrix umgeben sind, und wobei die Erosionsmatrix eines oder mehrere aus einem Quellmittel, einem Schmiermittel, einem Sprengmittel, einem Bindemittel und einem pharmazeutisch akzeptablen Hilfsstoff umfasst; und wobei das Stimulans Methylphenidat oder ein pharmazeutisch akzeptables Salz davon ist.

2. Darreichungsform nach Anspruch 1, wobei die erste Dosierungseinheit etwa 30 % bis etwa 40 % des gesamten Stimulans in der Darreichungsform freisetzt.

3. Darreichungsform nach Anspruch 1, wobei die erste Dosierungseinheit etwa 35 % bis etwa 40 % des gesamten Stimulans in der Darreichungsform freisetzt.

4. Darreichungsform nach Anspruch 1, wobei die zweite Darreichungseinheit etwa 40 % bis etwa 50 % des gesamten Stimulans in der Darreichungsform freisetzt.

5. Darreichungsform nach Anspruch 1, wobei die zweite Darreichungseinheit etwa 45 % bis etwa 50 % des gesamten Stimulans in der Darreichungsform freisetzt.

6. Darreichungsform nach Anspruch 1, wobei die dritte Dosierungseinheit etwa 10 % bis etwa 20 % des gesamten Stimulans in der Darreichungsform freisetzt.

7. Darreichungsform nach Anspruch 1, wobei die dritte Darreichungseinheit etwa 15 % bis etwa 20 % des gesamten Stimulans in der Darreichungsform freisetzt.

8. Einmal täglich verabreichte Darreichungsform eines der Ansprüche 1 bis 7 zur Anwendung bei der Behandlung einer Störung, eines Zustands oder einer Krankheit, für die ein Stimulans im Allgemeinen indiziert ist.

9. Einmal täglich anzuwendende Darreichungsform für die Anwendung nach Anspruch 8, wobei die Störung, der Zustand oder die Krankheit ADHS ist.

10. Dosierungsform mit pulsierender Freisetzung nach einem der Ansprüche 1, wobei
a) die erste Stimulansdosis im Wesentlichen unmittelbar in weniger als 10 Minuten nach der oralen Verabreichung der Darreichungsform an einen Patienten freigesetzt wird und/oder
b) die zweite Stimulansdosis bis etwa 2 bis etwa 5 Stunden nach der oralen Verabreichung der Darreichungsform an einen Patienten verzögert wird, und wobei die zweite Stimulansdosis über einen Zeitraum von etwa 45 bis 90 Minuten freigesetzt wird und/oder
c) die dritte Stimulansdosis bis etwa 6 bis etwa 8 Stunden nach der oralen Verabreichung der Darreichungsform an einen Patienten verzögert wird.

11. Darreichungsform nach Anspruch 10, wobei die zweite Stimulansdosis bis etwa 3 bis etwa 4 Stunden nach der oralen Verabreichung der Darreichungsform an einen Patienten verzögert wird.

12. Darreichungsform nach Anspruch 10, wobei die zweite Stimulansdosis über einen Zeitraum von etwa 60 Minuten bis etwa 90 Minuten freigesetzt wird.

## Revendications

1. Forme posologique à libération pulsatile pour l'administration orale d'un stimulant, comprenant
(a) une première unité posologique comprenant une première dose de stimulant qui est libérée essentiellement immédiatement après l'administration orale de la forme posologique à un patient ;
(b) une deuxième unité posologique comprenant une deuxième dose de stimulant, la deuxième dose de stimulant étant retardée d'environ 2 à environ 5 heures après l'administration de la forme posologique à un patient ; et
(c) une troisième unité posologique comprenant une troisième dose de stimulant, la troisième dose de stimulant étant retardée jusqu'à environ 5 à environ 9 heures après l'administration orale de la forme posologique à un patient ;
dans laquelle la forme posologique est un comprimé, et dans laquelle les unités posologiques sont incorporées à des positions spécifiques et contrôlées dans la forme posologique finie,
dans laquelle les première, deuxième et troisième unités posologiques comprennent chacune un noyau au moins partiellement entouré d'une matrice d'érosion,
dans laquelle le noyau de la première unité posologique est partiellement entouré d'une matrice d'érosion sur un bord de la première unité posologique, de telle sorte qu'au moins un bord extérieur de la première unité posologique n'est pas entouré par la matrice d'érosion et que les noyaux des deuxième et troisième unités posologiques sont entièrement entourés par la matrice d'érosion,
et dans laquelle la matrice d'érosion comprend un ou plusieurs éléments parmi un agent gonflant, un lubrifiantf, un délitant, un liant et un excipient pharmaceutiquement acceptable ; et
dans laquelle le stimulant est le méthylphénidate ou un sel pharmaceutiquement acceptable de celui-ci.

2. Forme posologique selon la revendication 1, dans laquelle la première unité posologique libère environ 30 % à environ 40 % de la quantité totale de stimulant dans la forme posologique.

3. Forme posologique selon la revendication 1, dans laquelle la première unité posologique libère environ 35 % à environ 40 % de la quantité totale de stimulant dans la forme posologique.

4. Forme posologique selon la revendication 1, dans laquelle la deuxième unité posologique libère environ 40 % à environ 50 % de la quantité totale de stimulant dans la forme posologique.

5. Forme posologique selon la revendication 1, dans laquelle la deuxième unité posologique libère environ 45 % à environ 50 % de la quantité totale de stimulant dans la forme posologique.

6. Forme posologique selon la revendication 1, dans laquelle la troisième unité posologique libère environ 10 % à environ 20 % de la quantité totale de stimulant dans la forme posologique.

7. Forme posologique selon la revendication 1, dans laquelle la troisième unité posologique libère environ 15 % à environ 20 % de la quantité totale de stimulant dans la forme posologique.

8. Forme posologique à prise unique quotidienne selon l'une quelconque des revendications 1 à 7 pour le traitement d'un trouble, d'un état ou d'une maladie pour lequel/laquelle un stimulant est généralement indiqué.

9. Forme posologique à prise unique quotidienne pour l'utilisation selon la revendication 8, dans laquelle le trouble, l'état ou la maladie est le TDAH.

10. Forme posologique à libération pulsatile selon l'une quelconque des revendications 1, dans laquelle
a) la première dose de stimulant est libérée essentiellement immédiatement, en moins de 10 minutes, après l'administration orale de la forme posologique à un patient et/ou
b) la deuxième dose de stimulant est retardée jusqu'à environ 2 à environ 5 heures après l'administration orale de la forme posologique à un patient, et dans laquelle la deuxième dose de stimulant est libérée pendant un espace de temps d'environ 45 à 90 minutes et/ou
c) la troisième dose de stimulant est retardée jusqu'à environ 6 à environ 8 heures après l'administration orale de la forme posologique à un patient.

11. Forme posologique selon la revendication 10, dans laquelle la deuxième dose de stimulant est retardée jusqu'à environ 3 à environ 4 heures après l'administration orale de la forme posologique à un patient.

12. Forme posologique selon la revendication 10, dans laquelle la deuxième dose de stimulant est libérée pendant un espace de temps d'environ 60 minutes à environ 90 minutes.
